Europäisches Patentamt

European Patent Office

Office européen des brevets

(19)

(11) Numéro de publication: **0 338 891**
**A1**

(12) **DEMANDE DE BREVET EUROPEEN**

(21) Numéro de dépôt: **89401000.8**

(22) Date de dépôt: **12.04.89**

(51) Int. Cl.⁴: **C 07 C 25/18**
C 07 C 17/12

(30) Priorité: **22.04.88 FR 8805330**

(43) Date de publication de la demande:
**25.10.89 Bulletin 89/43**

(84) Etats contractants désignés:
**AT BE CH DE ES FR GB GR IT LI LU NL SE**

(71) Demandeur: **RHONE-POULENC CHIMIE**
**25, quai Paul Doumer**
**F-92408 Courbevoie Cédex (FR)**

(72) Inventeur: **Nonn, Alain**
**11, allée de la gravière**
**F-69110 Sainte Foy Les Lyon (FR)**

(74) Mandataire: **Dubruc, Philippe et al**
**RHONE-POULENC CHIMIE Service Brevets Chimie 25,**
**quai Paul-Doumer**
**F-92408 Courbevoie Cédex (FR)**

(54) **Procédé de préparation du dibromo-4,4'biphenyle en milieu acide fort.**

(57) La présente invention concerne la préparation du dibromo-4,4' biphényle.

Selon l'invention, on fait réagir le biphényle ovec du brome dans un milieu comprenant un acide de pKa au plus égal à 4.

EP 0 338 891 A1

Bundesdruckerei Berlin

**Description**

## PROCEDE DE PREPARATION DU DIBROMO-4,4' BIPHENYLE EN MILIEU ACIDE FORT

La présente invention concerne la préparation du dibromo-4,4' biphényle.

Le dibromo-4,4' biphényle est un composé donnant accès par hydrolyse au dihydroxy-4,4' biphényle, ce dernier produit étant un monomère pour polymères thermotropes.

Il existe déjà plusieurs procédés de préparation du dibromo-4,4' biphényle. Toutefois, le besoin s'est fait sentir d'un procédé permettant d'activer la réaction de bromation et d'accroître éventuellement la sélectivité en dibromo-4,4' biphényle.

L'objet principal de l'invention est donc la mise au point d'un tel procédé.

Dans ce but, le procédé selon l'invention, de préparation du dibromo-4,4' biphényle est caractérisé en ce qu'on fait réagir le biphényle avec du brome dans un milieu comprenant au moins un acide de pKa au plus égal à 4.

Le procédé de l'invention permet, à température ambiante ou voisine de l'ambiante, d'obtenir des rendements qui peuvent être supérieurs à 85 %.

D'autres détails et caractéristiques de l'invention seront mieux compris à la lecture de la description et d'exemples concrets mais non limitatifs qui vont suivre.

La caractéristique essentielle de l'invention réside dans le fait que la réaction de bromation est conduite dans un milieu comprenant un acide fort, plus précisément un acide dont le pKa est au plus égal a 4.

Selon un mode de réalisation préférentiel, on utilise un acide dont le pKa est au plus égal à 1.

Généralement l'acide utilisé est choisi dans le groupe comprenant les acides carboxyliques ou sulfoniques.

On notera aussi que l'on peut employer les acides précités substitués par un ou plusieurs halogènes, notamment ceux substitués par le fluor.

On peut citer à titre d'exemples les acides carboxyliques aliphatiques notamment saturés ou aromatiques.

On peut utiliser bien entendu des mélanges d'acides.

D'autre part selon une autre variante de l'invention, il est possible de réaliser la bromation dans un milieu comprenant un solvant et un acide tel que défini ci-dessus.

Généralement, ce solvant est choisi dans le groupe comprenant les solvants hydrocarbures aliphatiques, cycliques, aromatiques et leurs équivalents halogénés et les solvants nitrés. On pourra aussi utiliser les éthers.

On peut citer à titre d'exemple le tétrachlorure de carbone, le dichlorométhane, le dichloro-1,2 éthane, l'o-dichlorobenzène, le nitrobenzène.

Dans le cas d'un milieu solvant-acide, la proportion d'acide par rapport au solvant peut varier dans toute proportion. Elle est généralement d'au moins 5 % en volume pouur obtenir une cinétique acceptable.

La réaction se fait à une température généralement comprise entre 10 et 75°C plus particulièrement entre 20 et 50°C.

La concentration en biphényle dans le milieu varie habituellement entre 0,1 et 3 moles/l et de préférence entre 1 et 2 moles/l.

Par rapport à la stoechiométrie on utilise en principe un excès de brome variant entre 0 et 20 %.

Le produit formé bromé pourra enfin être séparé du mélange réactionnel par tout moyen connu.

Des exemples vont maintenant être donnés.

EXEMPLE 1

Dans un réacteur de 250 ml muni d'un agitateur magnétique, d'un réfrigérant, d'une ampoule de coulée et d'un thermomètre, on charge :

15,4 g de biphényle (0,1 mole)

100 ml d'acide

A température ambiante, on coule en 10 minutes le brome (35,2 g - 0,22 mole) puis on agite pendant un temps variable toujours à température ambiante.

En fin de réaction, l'excès de brome est détruit par du sulfite ou du bisulfite de sodium. On ajoute la quantité suffisante d'eau et de dichlorométhane pour obtenir une décantation.

La phase organique est lavée, séchée et diluée à un volume donné pour être analysée en CPG.

Les autres conditions opératoires et résultats sont donnés dans le tableau ci-dessous.

| Acide | pKa | Durée (heure) | Taux de transformation | Rendement % en poids | | |
|---|---|---|---|---|---|---|
| | | | | Bromo-4 biphényle | di-Bromo-2,4' biphényle | di-Bromo-4,4' biphényle |
| HCO₂H | 3,75 | 8 | 97,5 % | 44,4 | 2,1 | 49,1 |
| CF₃CO₂H - | 0,3 | 8 | 100 % | 4,8 | 8,7 | 88,2 |
| CF₃SO₃H - | 14 | 5 | 100 % | 11,7 | 7,4 | 73,1 |

EXEMPLE 2 comparatif

On procède de la même manière que dans l'exemple 1 mais en utilisant l'acide acétique. Les résultats sont donnés ci dessous.

| Acide | pKa | Durée (heure) | Rendement % | |
|---|---|---|---|---|
| | | | di-Bromo-4,4' biphényle | Bromo-4 biphényle |
| AcOH | 4,75 | 29 | 15 | 68 |

EXEMPLE 3

Cet exemple illustre le mode de réalisation dans lequel le milieu reactionnel comprend un solvant et un acide.

On procède comme dans les exemples précédents en chargeant 9,10 g (0,059 mole) de biphényle, 53 ml de dichlorométhane et 5,9 ml d'acide trifluorométhane sulfonique.

Le brome (20,75 g soit 0,13 mole) est coulé en 18 minutes à température ambiante. Les rendements sont donnés ci-dessous en % en poids.

| | Temps de réaction | |
|---|---|---|
| | 5 h | 29 h |
| Bromo-4 biphényle | 0,4 % | 0,2 % |
| di-Bromo-2,4' biphényle | 5,6 % | 4,5 % |
| di-Bromo-4,4' biphényle | 76,8 % | 90,7 % |

Bien entendu, l'invention n'est nullement limitée aux modes de réalisation décrits qui n'ont été donnés qu'à titres d'exemples. En particulier, elle comprend tous les moyens constituant des équivalents techniques des moyens décrits ainsi que leurs combinaisons si celles-ci sont mises en oeuvre dans le cadre de la protection comme revendiquée.

**Revendications**

1. Procédé de préparation du dibromo-4,4' biphényle caractérisé en ce qu'on fait réagir le biphényle avec du brome dans un milieu comprenant un acide de pKa au plus égal à 4.

2. Procédé selon la revendication 1, caractérisé en ce que l'acide précité présente un pKa au plus égal à 1.

3. Procédé selon l'une quelconque des revendications précédentes caractérisé en ce qu'on choisit l'acide précité dans le groupe comprenant les acides carboxyliques ou sulfoniques.

4. Procédé selon la revendication 3 caractérisé en ce qu'on utilise un acide substitué par un ou des halogènes.

5. Procédé selon l'une des revendications précédentes caractérisé en ce que le milieu comprend un solvant en plus de l'acide.

6. Procédé selon la revendication 5 caractérisé en ce que le solvant est choisi dans le groupe comprenant les solvants hydrocarbures aliphatiques, cycliques, aromatiques et leurs équivalents halogénés, les solvants nitrés, les éthers.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | US-A-3 518 316  (CUMBO) <br> * colonne 2, lignes 16-20; revendication 1 * <br> --- | 1-5 | C 07 C  25/18 <br> C 07 C  17/12 |
| A | BEILSTEINS HANDBUCH DER ORGANISCHEN CHEMIE <br> 4ième édition, tome 5, Verlag Julius Springer Berlin * I. supplément (1930), page 273g; II. supplément (1943), page 485j * <br> --- | 1 | |
| A | PATENT ABSTRACTS OF JAPAN <br> tome 4, no. 101 (C-19)(583), 19 juillet 1980; & JP - A - 55 64532 (HOBOGAYA KAGAKU) 15.05.80 <br> --- | 1 | |
| A | HOUBEN-WEYL: "METHODEN DER ORGANISCHEN CHEMIE" <br> 4ième édition, tome V/4, 1960, Georg Thieme Verlag, Stuttgart * page 235, lignes 17-19 * <br> ----- | 1 | |

**DOMAINES TECHNIQUES RECHERCHES (Int. Cl.4 )**

C 07 C  17/00
C 07 C  25/00

Le présent rapport a été établi pour toutes les revendications

| Lieu de la recherche | Date d'achèvement de la recherche | Examinateur |
|---|---|---|
| BERLIN | 14-07-1989 | PROBERT C.L. |

EPO FORM 1503 03.82 (P0402)